# EUROPÄISCHE PATENTSCHRIFT

(11) **EP 3 228 280 B1**
(45) Veröffentlichungstag und Bekanntmachung des Hinweises auf die Patenterteilung: **10.07.2019**
(21) Anmeldenummer: 16163669.1
(22) Anmeldetag: 04.04.2016
(51) Int. Cl.: A61B 17/56, A61F 2/32

(54) **AM BECKENKNOCHEN MONTIERBARE ABSTÜTZVORRICHTUNG**
SUPPORTING DEVICE MOUNTABLE AT THE PELVIC BONE
DISPOSITIF D'APPUI A MONTER SUR L'OS DE LA HANCHE

(43) Veröffentlichungstag der Anmeldung: 11.10.2017
(73) Patentinhaber: Baumgart, Rainer, 80539 München (DE)
(72) Erfinder: Baumgart, Rainer, 80539 München (DE)
(74) Vertreter: Lambacher, Michael

(56) Entgegenhaltungen:
- EP-A2- 1 442 727
- DE-U1- 29 513 694
- US-A- 2 947 308
- US-A- 5 702 477
- US-A1- 2002 165 615
- US-A1- 2011 264 216
- US-A1- 2012 253 414
- US-A1- 2014 257 501

## Beschreibung

Die Erfindung betrifft eine am Beckenknochen montierbare Abstützvorrichtung nach dem Oberbegriff des Patentanspruchs 1 sowie eine die Abstützvorrichtung aufweisende Vorrichtung zur Dehnung von Weichteilen im Hüftbereich.

Aus der US 2012/0253414 A1 ist eine Entlastungsvorrichtung für Gelenke bekannt. Gemäß einer Ausführungsform umfasst die Vorrichtung eine Basis und einen über ein Kugelgelenk mit der Basis beweglich gekoppelten starren Stab. Die Vorrichtung ist außerhalb des noch erhaltenen Gelenkes angebracht.

Der endoprothetische Hüftgelenksersatz ist ein verbreitetes operatives Verfahren, wenn das menschliche Hüftgelenk verschlissen ist und starke Schmerzen resultieren. Hierbei wird an der gleichen Stelle, wo das natürliche Hüftgelenk platziert ist, eine Schale implantiert, in der ein künstlicher, runder Gelenkkopf, der mit einem Schaft in dem Oberschenkelknochen verankert ist, artikulieren kann. Derartige Prothesen sind aus der US 2,947,308 A und der DE 295 13 694 U1 bekannt.

Wenn aufgrund einer fehl angelegten (dysplastischen) Pfanne oder nach einem Unfallereignis oder nach einer Tumorresektion das Hüftgelenk kopfwärts (nach cranial) verschoben ist, resultiert regelhaft einerseits eine Beinverkürzung und andererseits meist eine deutliche Bewegungseinschränkung, wobei sich um den Hüftkopf meist ein sogenanntes Neo-Gelenk ausbildet, das eine belastungsfähige Abstützung gewährleistet.

Bei einer derartigen Ausgangssituation, in der das Hüftgelenk kopfwärts ausgewandert ist, ist es bisher nur sehr unbefriedigend möglich, ein künstliches Hüftgelenk zu implantieren, da einerseits die Implantation eines Kunstgelenkes im Bereich der Beckenschaufel sehr schlechte Langzeitergebnisse liefert und andererseits die Platzierung des Kunstgelenkes in anatomisch regelrechter Position nur möglich ist, wenn der Oberschenkelknochen fußwärts gezogen und damit die Weichteile gedehnt werden oder eine Verkürzung des Oberschenkelknochens vorgenommen wird. Eine akute Dehnung von mehr als etwa 2 cm führt regelhaft zu einer Schädigung des Nervus Ischiadikus und damit zu Funktionsausfällen. Bei einer Kürzung des Oberschenkelknochens verbleibt meist eine erhebliche Beinlängendifferenz und zudem eine Funktionsstörung, da wichtige Muskelansätze geopfert werden müssen.

Kontinuierliche Verlängerungen, die das Risiko einer Nervenschädigung verringern, waren früher nur mit externen Fixateuren, die zum einen am Becken und zum anderen am Oberschenkelknochen befestigt werden, möglich. Diese Fixateure sind jedoch extrem belastend für den Patienten und es besteht ein erhebliches Infektions- oder zumindest Kontaminationsrisiko, was im Hinblick auf die darauf folgende Hüftendoprothese besonders problematisch ist.

Es ist bekannt, Beinverlängerungen mit voll-implantierbaren, motorisierten Distraktionsmarknägeln durchzuführen, wie sie beispielsweise in der EP 1 371 346 A1 beschrieben sind. Ein weiteres Anwendungsgebiet dieser Distraktionsmarknägel ist es nun, nicht nur einen Knochen zu verlängern, sondern auch die Weichteile zu dehnen, um z. B. wie in der zuvor geschilderten Situation den Oberschenkelknochen fußwärts zu verlagern, um anschließend eine Hüftgelenksendoprothese in regelrechter Position implantieren zu können.

Erste klinische Anwendungen hierzu, die in *"*J Bone Joint Surg Vol.87-B, No.4, April 2005" beschrieben sind, gab es bereits vor über 10 Jahren. Die bei dieser Anwendung verwendete Abstützvorrichtung umfasst eine Grundplatte mit einer Aufnahmeeinrichtung, die einen Schlitz aufweist, in den das Stützende des Teleskopteils des Distraktionsmarknagels eingeführt wurde, wobei die Bewegung des Stützendes stark eingeschränkt und hinsichtlich der Hebelwirkungen unkontrolliert stattfindet.

Das Problem liegt somit in der Abstützung des Stützendes des Teleskopteils auf der proximalen beckenzugewandten Seite, da hier einerseits ausreichend Axialkräfte aufgenommen werden müssen, andererseits aber der Bewegungsumfang so wenig wie möglich eingeschränkt sein darf, da ansonsten durch die Hebelkräfte des Beines die Befestigung der Vorrichtung ausbrechen würde.

Die der Erfindung zugrunde liegende Aufgabe liegt nun darin, eine Abstützvorrichtung zu realisieren, die den anatomischen Gegebenheiten an der Außenseite des Beckenknochens angepasst ist und im unteren Bereich, wo genügend Knochenmasse am Pfannendach vorliegt, so befestigt wird, dass die hier auftretenden Zug- und Scherkräfte aufgenommen werden, während die resultierenden Druckkräfte im oberen Bereich flächig übertragen werden. Weiterhin gilt es einerseits, eine weitestgehende Bewegungsfreiheit des Beines zu ermöglichen, damit die Hebelkräfte aufgrund der Länge des Beines (ca. 1 m) die Fixierungsschrauben der Vorrichtung nicht aus dem Beckenknochen ziehen und damit zu einer Lockerung führen und andererseits keine Luxation (Entfernen der Gelenkpartner voneinander) eintritt.

Die der Erfindung zugrunde liegende Aufgabe wird durch eine Abstützvorrichtung mit den Merkmalen des Patentanspruchs 1 gelöst. Vorteilhafte Ausführungsformen der erfindungsgemäßen Abstützvorrichtung sind Gegenstand der Patentansprüche 2 bis 6.

Eine mit der Abstützvorrichtung versehene Vorrichtung zur Dehnung von Weichteilen im Hüftbereich umfasst die Merkmale der Patentansprüche 7 und 8.

Bei der erfindungsgemäßen am Beckenknochen montierbaren Abstützvorrichtung umfasst die Aufnahmeeinrichtung einen Kugelsitz mit einer eine Kugelsitzfläche bildenden Kavität zur Aufnahme einer an dem Stützende des Teleskopteils vorgesehenen Kugel. Der Innenradius der Kugelsitzfläche entspricht dem Außenradius der Kugel. Der Kugelsitz ist so ausgebildet und angeordnet, dass der Teleskopteil eine für ein weitgehend normales Bewegungsmuster des Oberschenkelknochens ausreichende Bewegung durchführen und nicht aus dem Kugelsitz luxieren kann, wenn der Verankerungsteil in dem Oberschenkelknochen implantiert ist.

Die ausreichende Bewegung wird beispielsweise gewährleistet, wenn sich die Längsachse des Teleskopteils innerhalb eines Bereichs eines gedachten Kegels mit einem Kegelwinkel von ca. 25-45°, vorzugsweise ca. 35° bewegen kann, wenn der Verankerungsteil im Oberschenkelknochen implantiert ist und die Abstützvorrichtung am Beckenknochen montiert ist.

Vorzugsweise sind in der Grundplatte bezüglich der Aufnahmeeinrichtung distal wenigstens zwei im Abstand zueinander angeordnete Durchgangsöffnungen vorgesehen. An diesen Durchgangsöffnungen wird die Abstützvorrichtung an dem Beckenknochen mittels Spongiosaschrauben befestigt. Diese Befestigung ist ausreichend, um Zugkräfte aufzunehmen zu können und Druckkräfte auf den Beckenknochen zu übertragen. Befestigungsöffnungen proximal der Aufnahmeeinrichtung sind somit nicht erforderlich, sie können aber zur weiteren Stabilisierung vorgesehen werden. Der proximale Rand der Grundplatte kann unter die an dem Beckenknochen anliegende Muskulatur geschoben werden, ohne dass hier eine zusätzliche Befestigung erforderlich ist, da in diesem Bereich in erster Linie Druckkräfte auftreten.

Erfindungsgemäß umfasst der Kugelsitz eine bevorzugt von einem Stützblock gebildete Basis mit einer Teilkavität mit einer ersten Teilkugelsitzfläche, die so ausgebildet und angeordnet ist, dass die Kugel frei in die Teilkavität eingelegt werden kann. Eine Abdeckung mit einer zweiten geringen Teilkugelsitzfläche ist so befestigbar, dass die erste Teilkugelsitzfläche zur Bildung einer Gesamtkugelsitzfläche in die zweite Teilkugelsitzfläche übergeht, wobei die Gesamtkugelsitzfläche größer ist als die Halbkugelaußenfläche der Kugel. Die Abdeckung bildet hierdurch eine Hinterschneidung, die eine Luxation der Kugel aus der Kavität des Kugelsitzes verhindert.

Eine ausreichende Sicherheit ist in diesem Fall gewährleistet, wenn die Gesamtkugelsitzfläche 2% bis 5% größer ist als die Halbkugelaußenfläche.

Wenigstens muss der Innenumfang der Gesamtkugelsitzfläche an einer Stelle größer sein als der halbe Umfang der Kugel.

Da die Grundplatte an ihrem proximalen Rand abgeflacht ist, kann der proximale Rand der Grundplatte ohne Verletzungsgefahr unter die Muskulatur am Beckenknochen geschoben werden.

Zur Verminderung der Gleitreibung wird bevorzugt eine Kunststoffschale in die Kavität eingesetzt oder eine reibungsvermindernde Beschichtung auf der Kugel aufgebracht oder die Kavität mit reibungsverminderndem Material beschichtet.

Ein Ausführungsbeispiel der Erfindung wird nachstehend anhand von Zeichnungen näher erläutert. Es zeigen
Figur 1 eine perspektivische Ansicht einer erfindungsgemäßen Abstützvorrichtung;
Figur 2 eine Draufsicht auf die Abstützvorrichtung von Fig. 1;
Figur 3 einen medianen Querschnitt der Abstützvorrichtung von Fig. 1;
Figur 4 eine die Abstützvorrichtung aufweisende Vorrichtung zur Dehnung von Weichteilen im implantierten Zustand zu Beginn einer Behandlung;
Figur 5 die Vorrichtung von Figur 4 nach Dehnung der Weichteile gegen Ende einer Behandlung;

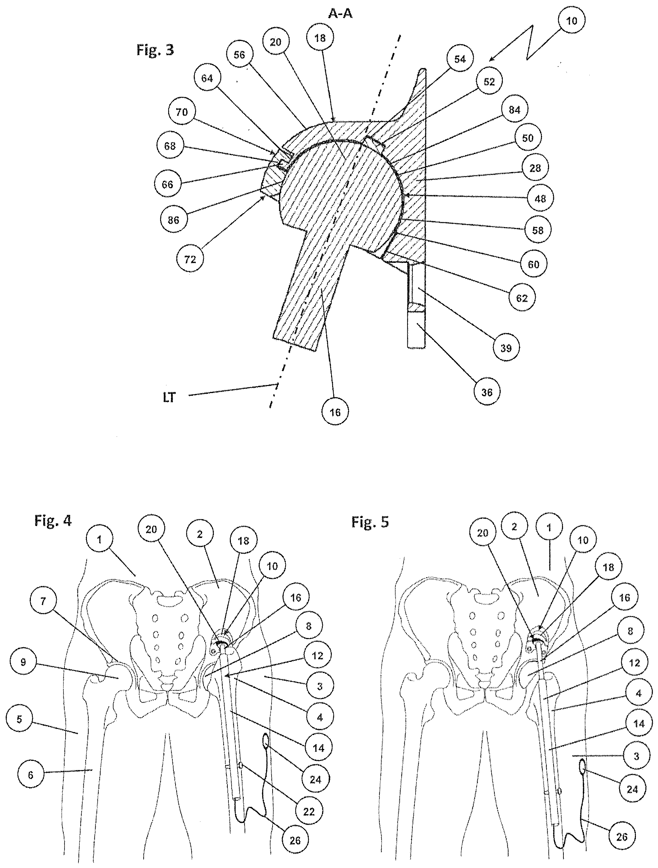

In den Figuren 4 und 5 ist schematisch der Skelettaufbau eines Patienten im Hüftbereich gezeigt, wobei in Figur 4 der linke Oberschenkel des Patienten kopfwärts (nach cranial) verschoben ist. Diese Verschiebung kann aus einer fehl angelegten Pfanne oder nach einem Unfallereignis oder nach einer Tumorresektion resultieren.

Im unteren Bereich des Torsos 1 ist das Becken 2 angeordnet. In Figur 4 ist der Gelenkkopf 9 am oberen Ende des Oberschenkelknochens 6 des rechten Oberschenkels 5 korrekt in der Hüftpfanne 7 angeordnet. Bei dem linken Oberschenkel 3 des Patienten ist der Oberschenkelknochen 4 bezüglich der Hüftpfanne 8 kopfwärts nach oben verschoben.

Um eine Verlagerung des Oberschenkelknochens 4 fußwärts zu ermöglichen und die entsprechenden Weichteile zu dehnen, ist in dem Oberschenkelknochen 4 ein Distraktionsmarknagel 12 implantiert, der einen in Längsrichtung des Oberschenkelknochens 4 in diesem implantierten Verankerungsteil 14 umfasst, der mittels wenigstens einer quer angeordneten Befestigungsschraube 22 an dem Oberschenkelknochen 4 befestigt ist. In dem Verankerungsteil 14 ist ein Teleskopteil 16 in Längsrichtung des Verankerungsteils 14 verschiebbar geführt. An dem proximalen, d.h. dem Torso 1 zugewandten Ende des Teleskopteils 16 ist eine Kugel 20 beispielsweise durch Kegelpresssitz befestigt, die in einer Aufnahmeeinrichtung 18 einer Abstützvorrichtung 10 gelenkig gelagert ist. Die Abstützeinrichtung 10 ist am Os llium des Beckens 2 montiert. Der Teleskopteil 16 kann mittels eines in dem Verankerungsteil 14 angeordneten Motors in proximaler Richtung verschoben werden. Zur Steuerung des Motors ist der Motor über ein Kabel 26 mit einer unter der Haut des Oberschenkels angeordneten Antenne 24 verbunden. Eine entsprechende Konstruktion eines Marknagels ist beispielsweise aus der EP 1 371 346 A1 bekannt.

Der Teleskopteil 16 wird schrittweise über einen längeren Zeitraum durch den Motor in proximaler Richtung verschoben. Der Teleskopteil 16 stützt sich dabei über die am proximalen Stützende des Teleskopteils 16 angeordnete Kugel 20, die in der Aufnahmeeinrichtung 18 gelenkig aufgenommen ist, an der Abstützeinrichtung 10 ab, die wiederum an dem Os llium des Beckens 2 montiert ist. Hierdurch wird der Oberschenkelknochen 4 in distaler Richtung, d.h. vom Torso 1 weg, verschoben, wobei gleichzeitig die Weichteile gedehnt werden. Ein solches Verfahren zur Dehnung der Weichteile ist in der Druckschrift "J Bone Joint Surg Vol.87-B, No.4, April 2005" beschrieben, so dass das Verfahren im Folgenden nicht weiter erläutert wird.

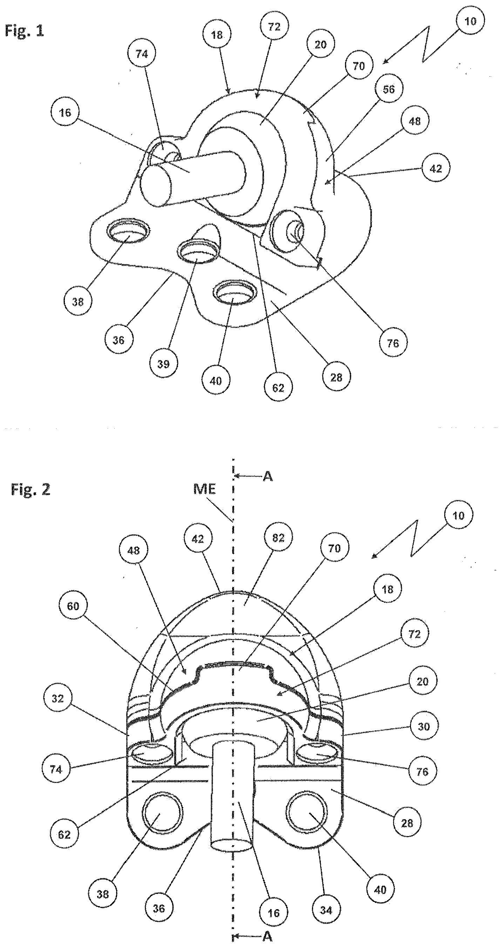

Der Aufbau der Abstützvorrichtung 10 und die Lagerung der Kugel 20 des Teleskopteils 16 in der Aufnahmeeinrichtung 18 ist in den Figuren 1 bis 3 im Detail gezeigt.

Die Abstützvorrichtung 10 ist bezüglich einer senkrecht zur Plattenebene verlaufenden Mittelebene ME symmetrisch ausgebildet. Im Folgenden werden bezüglich der Anordnung einzelner Elemente die Ausdrücke "proximal" und "distal" verwendet, wobei proximal dem Torso 1 zugewandt und distal von dem Torso 1 abgewandt bedeutet.

Die Abstützvorrichtung 10 umfasst eine Grundplatte 28, die zwei Seitenränder 30, 32 aufweist, die im distalen Abschnitt parallel zueinander und parallel zur Mittelebene ME verlaufen und anschließend in proximaler Richtung zur Mittelebene ME hin gekrümmt verlaufen, so dass sie eine abgerundete vordere Spitze 42 am Schnittpunkt mit der Mittelebene ME bilden. In dem distalen, insgesamt senkrecht zur Mittelebene ME verlaufenden Seitenrand 34 ist mittig eine Einbuchtung 36 ausgebildet. Beidseitig der Mittellinie ME gehen angrenzend an den distalen Seitenrand 34 zwei Durchgangsöffnungen 38, 40 durch die Grundplatte 28 hindurch. Eine weitere Durchgangsöffnung 39 ist mittig zwischen den Durchgangsöffnungen 38 und 40 ausgebildet.

Proximal zu den Durchgangsöffnungen 38, 39, 40 ist auf der Grundplatte 28 eine Aufnahmeeinrichtung 18 vorgesehen, die einen Kugelsitz 48 umfasst, in dem eine Kavität 58 ausgebildet ist, in der die Kugel 20 am Stützende des Teleskopteils 16 aufgenommen ist.

Der Kugelsitz 48 besteht aus zwei Elementen, die jeweils eine Teilkugelsitzfläche bilden. Ein Stützblock 56 erstreckt sich von der Oberseite der Grundplatte 28 nach oben. In dem Stützblock 56 ist eine Teilkavität 84 ausgebildet, die eine erste Teilkugelsitzfläche definiert. Die Teilkavität 84 ist in distaler Richtung hin offen und wird durch einen Stirnrand 60 begrenzt, der senkrecht zur Mittelebene ME verläuft.

Wie es in Figur 3 zu erkennen ist, ist der Kugelsektor des sphärischen Abschnittes der Teilkavität 84 etwas kleiner als der Halbkugelsektor der Kugel 20 des Teleskopteils 16. Von dem unteren Ende des sphärischen Teils der Teilkavität 84 erstreckt sich die Oberfläche einer Schrägstufe 62 im Wesentlichen tangential in distaler Richtung schräg nach oben.

Die Teilkavität 84 des Stützblocks 56 ist daher so ausgebildet, dass die Kugel 20 des Teleskopteils 16 in die Teilkavität 84 eingelegt werden kann, wenn keine Abdeckung 72 angebracht ist, ohne das die Kugel akut nach distal verschoben werden muss.

Die Abdeckung 72 wird an der Stirnseite des Stützblocks 56 angebracht. Die Abdeckung 72 ist im Wesentlichen U-förmig ausgebildet und liegt mit den freien Enden ihrer Schenkel auf der Schrägstufe 62 auf. An den Enden der beiden freien Schenkel ist jeweils eine Durchgangsöffnung 74 und 76 ausgebildet, durch die Befestigungsschrauben hindurchgehen können, die die Abdeckung 72 an dem Stützblock 56 befestigen. Die Innenseite der Abdeckung 72 bildet im oberen, bogenförmigen Bereich eine zweite Teilkugelsitzfläche 86, die bündig an die Teilkugelsitzfläche der Kavität 58 anschließt, wodurch die Gesamtkugelsitzfläche so erhöht wird, dass sie größer ist als die Halbkugelfläche der Kugel 20. Mit anderen Worten wird durch die Abdeckung 72 eine Hinterschneidung gebildet, die verhindert, dass die Kugel 20 aus dem Kugelsitz 48 luxieren kann, wenn die Abdeckung 72 an dem Stützblock 56 angebracht ist.

Für eine feste Verankerung der Abdeckung 72 an dem Stützblock 56 ist im oberen Abschnitt des Stützblocks 56 angrenzend an den Stirnrand 60 eine Nut 64 ausgebildet, wodurch am Rand 60 ein Randvorsprung 66 definiert wird. Gleichzeitig ist in der Abdeckung 72 ein Eingriffsschenkel 70 mit einer Eingriffsnut 68 ausgebildet, die über den Randvorsprung 66 in die Nut 64 eingreift. Durch die Bildung der Eingriffsnut 68 wird somit am proximalen Rand der Abdeckung 72 ein Eingriffsschenkel 70 gebildet, der in die Nut 64 eingreift. Hierdurch wird eine formschlüssige Verbindung der Abdeckung 72 mit dem Stützblock 56 geschaffen, wenn die Abdeckung 72 mittels durch die Durchgangsöffnungen 74, 76 hindurchgehender Befestigungsschrauben an dem Stützblock 56 befestigt wird.

Um die Gleitreibung zwischen der Kugel 20 in der Kavität 58 des Kugelsitzes 48 zu verringern, ist in der Kavität 58 eine Kunststoffschale 50 angeordnet, die an der Sitzfläche anliegt. An der Kunststoffschale 50 ist im proximalen Bereich ein Vorsprung 52 ausgebildet, der in eine Ausnehmung 54 in der Kavität 58 eingreift, wodurch eine sphärische Verschiebung der Kunststoffschale verhindert wird und ein sicherer Halt der Kunststoffschale 50 in der Kavität 58 gewährleistet ist. Die Kunststoffschale 50 bildet somit die tatsächliche Kavität, deren Innendurchmesser dem Außendurchmesser der Kugel 20 entspricht.

Der Kugelsitz 48 ist insgesamt so ausgebildet, dass sich die Längsachse LT des Teleskopteils 16 innerhalb eines Bereichs eines gedachten Kegels mit einem Kegelwinkel von 25-45°, vorzugsweise 35°, bewegen kann, wenn der Verankerungsteil 14 im Oberschenkelknochen 4 implantiert ist und die Abstützvorrichtung 10 am Beckenknochen 2 montiert ist.

Proximal der Aufnahmevorrichtung 18 ist die Grundplatte 28 zur Spitze 42 hin abgeflacht, wodurch die Spitze 42 unter die dem Beckenknochen anliegende Muskulatur ohne Verletzungsgefahr geschoben werden kann.

Die Grundplatte 28 bildet mit der Aufnahmeeinrichtung 18 bis auf die Abdeckung 72 bevorzugt ein Teil, wobei alle Ränder und Kanten abgerundet sind, um eine Verletzungsgefahr zu minimieren.

Die Grundplatte 28 mit der Aufnahmevorrichtung 18 und der Abdeckung 72 ist vorzugsweise in Stahl oder Titan ausgebildet. Die Kunststoffschale 50 ist vorzugsweise aus PTFE oder PEEK ausgebildet.

### Bezugszeichenliste

1 - Rumpf
2 - Beckenknochen
3 - linker Oberschenkel
4 - linker Oberschenkelknochen
5 - rechter Oberschenkel
6 - rechter Oberschenkelknochen
7 - Hüftpfanne rechts
8 - Hüftpfanne links
9 - Gelenkkopf
10 - Abstützvorrichtung
12 - Distraktionsmarknagel
14 - Verankerungsteil
16 - Teleskopteil
18 - Aufnahmeeinrichtung
20 - Kugel
22 - Schraube
24 - Antenne
26 - Kabel
28 - Grundplatte
30 - rechter Seitenrand
32 - linker Seitenrand
34 - distaler Seitenrand
36 - Einbuchtung
38 - Durchgangsöffnung
39 - Durchgangsöffnung
40 - Durchgangsöffnung
42 - Spitze
48 - Kugelsitz
50 - Kunststoffschale
52 - Vorsprung
54 - Ausnehmung
56 - Stützblock
58 - Kavität
60 - Stirnrand
62 - Schrägstufe
64 - Nut
66 - Randvorsprung
68 - Eingriffsnut
70 - Eingriffsschenkel
72 - Abdeckung
74 - Durchgangsöffnung
76 - Durchgangsöffnung
82 - Abflachung
84 - Teilkavität
86 - Teilkugelsitzfläche

## Patentansprüche

1. Am Beckenknochen (2) montierbare Abstützvorrichtung (10) zur Abstützung eines Stützendes eines proximalen Teleskopteils (16) eines mit einem distalen Verankerungsteil (14) in einen Oberschenkelknochen (4) implantierbaren Distraktionsmarknagels (12), mit
- einer am Beckenknochen (2) befestigbaren Grundplatte (28) und
- einer auf einer dem Beckenknochen abgewandten Seite der Grundplatte (28) vorgesehenen Aufnahmeeinrichtung (18) zur beweglichen Aufnahme des Stützendes des Teleskopteils (16),
**dadurch gekennzeichnet, dass**
- die Aufnahmeeinrichtung (18) einen Kugelsitz (48) mit einer eine Kugelsitzfläche bildenden Kavität (58) zur Aufnahme einer an dem Stützende des Teleskopteils (16) vorgesehenen Kugel (20) umfasst, wobei der Innenradius der Kugelsitzfläche dem Außenradius der aufnehmbaren Kugel (20) entspricht, wobei der Kugelsitz (48) eine Basis (56) mit einer Teilkavität (84) mit einer ersten Teilkugelsitzfläche umfasst, die so ausgebildet und angeordnet ist, dass die Kugel (20) frei in die Teilkavität (84) eingelegt werden kann, und eine Abdeckung (72) mit einer zweiten Teilkugelsitzfläche umfasst, die so befestigbar ist, dass die erste Teilkugelsitzfläche zur Bildung einer Gesamtkugelsitzfläche in die zweite Teilkugelsitzfläche übergeht, wobei die Gesamtkugelsitzfläche größer ist als die Halbkugelaußenfläche der Kugel (20), und
- die Grundplatte (28) an ihrem proximalen Rand abgeflacht ausgebildet ist.

2. Abstützvorrichtung (10) nach Anspruch 1, **dadurch gekennzeichnet, dass** in der Grundplatte (28) bezüglich der Aufnahmeeinrichtung (18) distal wenigstens zwei im Abstand zueinander angeordnete Durchgangsöffnungen (38, 39, 40) vorgesehen sind.

3. Abstützvorrichtung (10) nach einem der vorhergehenden Ansprüche, **dadurch gekennzeichnet, dass** die Abstützvorrichtung (10) eine Schrägstufe (62) umfasst, deren Oberfläche sich von einem unterem Ende der Teilkavität (84) im Wesentlichen tangential in distaler Richtung nach oben erstreckt.

4. Abstützvorrichtung (10) nach Anspruch 3, **dadurch gekennzeichnet, dass** die Abdeckung (72) im Wesentlichen U-förmig ausgebildet ist und im befestigten Zustand mit den freien Enden ihrer Schenkel auf der Schrägstufe (62) aufliegt.

5. Abstützvorrichtung (10) nach Anspruch 1, **dadurch gekennzeichnet, dass** die Gesamtkugelsitzfläche 5% bis 10% größer ist als die Halbkugelaußenfläche.

6. Abstützvorrichtung (10) nach einem der vorhergehenden Ansprüche, **dadurch gekennzeichnet, dass** zur Verminderung der Gleitreibung eine Kunststoffschale (50) in die Kavität (58) eingesetzt ist oder eine reibungsvermindernde Beschichtung auf der Kugel (20) aufgebracht ist oder die Kavität (58) mit reibungsverminderndem Material beschichtet ist.

7. Vorrichtung zur Dehnung von Weichteilen im Hüftbereich, umfassend:
einen Distraktionsmarknagel (12), der einen in einen Oberschenkelknochen (4) implantierbaren, distalen Verankerungsteil (14) und einen bezüglich des Verankerungsteils (14) in Längsrichtung verfahrbaren, proximalen Teleskopteil (16) mit einem proximalen Stützende aufweist, an dem eine Kugel (20) angebracht ist; und
die Abstützvorrichtung (10) nach einem der Ansprüche 1 bis 6 zur Abstützung des Stützendes des proximalen Teleskopteils (16).

8. Vorrichtung nach Anspruch 7, wobei der Kugelsitz (48) der Abstützvorrichtung (10) und der Distraktionsmarknagel (12) so ausgebildet sind, dass bei im Kugelsitz(48) aufgenommener Kugel (20) sich die Längsachse (LT) des Teleskopteils (16) des Distraktionsmarknagels (12) innerhalb eines Bereichs eines gedachten Kegels mit einem Kegelwinkel von 25-45°, vorzugsweise ca. 35° bewegen kann, wenn der Verankerungsteil (14) im Oberschenkelknochen (4) implantiert ist und die Abstützvorrichtung (10) am Beckenknochen (2) montiert ist.

## Claims

1. A support apparatus (10) that can be mounted on the pelvic bone (2) to support a support end of a proximal telescoping part (16) of a distraction marrow nail (12) that can be implanted with a distal anchoring part (14) into a femur (4), comprising
- a base plate (28) that can be affixed to the pelvic bone (2), and
- an accommodating device (18) that is provided on a side of the base plate (28) facing away from the pelvic bone for movably accommodating the support end of the telescoping part (16),
**characterized in that**
- the accommodating device (18) comprises a ball seat (48) having a cavity (58) that forms a ball seat surface for accommodating a ball (20) provided at the support end of the telescoping part (16), wherein the inside radius of the ball seat surface corresponds to the outside radius of the ball (20) to be accommodated, wherein the ball seat (48) comprises a base (56) having a sub-cavity (84) with a first partial ball seat surface, which is configured and disposed in such a manner that the ball (20) can be freely laid into the sub-cavity (84), and comprises a covering (72) having a second partial ball seat surface, which can be affixed in such a manner that the first partial ball seat surface makes a transition into the second partial ball seat surface to form a total ball seat surface, wherein the total ball seat surface is larger than the external hemispherical surface of the ball (20), and
- the base plate (28) is flattened at its proximal edge.

2. The support apparatus (10) according to claim 1, **characterized in that** at least two passage openings (38, 39, 40) disposed at a distance from one another are provided in the base plate (28) distally with reference to the accommodating device (18).

3. The support apparatus (10) according to one of the preceding claims, **characterized in that** the support apparatus (10) comprises a slanted step (62), the surface of which extends from a lower end of the sub-cavity (84) essentially tangentially in the distal direction upwards.

4. The support apparatus (10) according to claim 3, **characterized in that** the covering (72) is essentially configured in U shape and when affixed lies on the slanted step (62) with the free ends of its shanks.

5. The support apparatus (10) according to claim 1, **characterized in that** the total ball seat surface is 5% to 10% larger than the external hemispherical surface.

6. The support apparatus (10) according to one of the preceding claims, **characterized in that** in order to reduce the slide friction, a plastic shell (50) is inserted into the cavity (58) or a friction-reducing coating is applied to the ball (20) or the cavity (58) is coated with friction-reducing material.

7. An apparatus for stretching soft tissue parts in the hip region, comprising:
a distraction marrow nail (12) that has a distal anchoring part (14) that can be implanted into a femur (4) and a proximal telescoping part (16) that can be moved in the longitudinal direction with reference to the anchoring part (14) and has a proximal support end, at which a ball (20) is attached, and
the support device (10) according to one of claims 1 to 6 to support the support end of the proximal telescoping part (16).

8. The apparatus according to claim 7, wherein the ball seat (48) of the support apparatus (10) and the distraction marrow nail (12) are configured in such a manner that when the ball (20) is accommodated in the ball seat (48) the longitudinal axis (LT) of the telescoping part (16) of the distraction marrow nail (12) can move within a range of an imaginary cone having a cone angle of 25-45°, preferably approximately 35°, when the distraction marrow nail (14) is implanted in the femur (4) and the support apparatus (10) is mounted on the pelvic bone (2).

## Revendications

1. Dispositif de support (10) à monter sur l'os du bassin (2) destiné à supporter une extrémité d'appui d'une pièce télescopique (16) proximale d'un clou médullaire de distraction (12) implantable dans un os de fémur (4) avec une pièce d'ancrage (14) distale, comprenant :
- une embase (28) pouvant être fixée à l'os du bassin (2), et
- un dispositif de logement (18) agencé sur un côté de l'embase (28) opposé à l'os du bassin pour recevoir de façon mobile l'extrémité d'appui de la pièce télescopique (16),
**caractérisé en ce que** :
- le dispositif de logement (18) comprend un logement sphérique (48) comportant une cavité (58) formant une surface de logement sphérique destinée à recevoir une tête sphérique (20) disposée sur l'extrémité d'appui de la pièce télescopique (16), dans lequel le rayon intérieur de la surface de logement sphérique correspond au rayon extérieur de la tête sphérique (20) pouvant être logée et le logement sphérique (48) comprend une base (56) comportant une cavité partielle (84) dotée d'une première surface partielle de logement sphérique, qui est configurée et agencée de manière à ce que la tête sphérique (20) puisse être insérée librement dans la cavité partielle (84) et comprend une coiffe (72) comportant une seconde surface partielle de logement sphérique, qui peut être fixée de manière à ce que la première surface partielle de logement sphérique se prolonge dans la seconde surface partielle de logement sphérique pour former une surface complète de logement sphérique, dans lequel la surface complète de logement sphérique est plus grande que la surface extérieure de la moitié de la tête sphérique (20), et
- l'embase (28) est configurée de manière à être aplatie à son bord proximal.

2. Dispositif de support (10) selon la revendication 1, **caractérisé en ce qu'**au moins deux orifices de passage (38, 39, 40) disposés à distance l'un de l'autre sont agencés dans l'embase (28) de façon distale par rapport au dispositif de logement (18).

3. Dispositif de support (10) selon l'une des revendications précédentes, **caractérisé en ce que** le dispositif de support (10) comprend un échelon incliné (62), dont la surface s'étend à partir de l'extrémité inférieure de la cavité partielle (84) essentiellement de façon tangentielle en direction distale vers le haut.

4. Dispositif de support (10) selon la revendication 3, **caractérisé en ce que** la coiffe (72) est configurée essentiellement en forme de U et repose avec les extrémités libres de ses branches sur l'échelon incliné (62) à l'état fixé.

5. Dispositif de support (10) selon la revendication 1, **caractérisé en ce que** la surface complète de logement sphérique est de 5 à 10 % plus grande que la surface extérieure de la demi-tête sphérique.

6. Dispositif de support (10) selon l'une des revendications précédentes, **caractérisé en ce que**, pour diminuer le frottement de glissement, une coque en matière plastique (50) est insérée dans la cavité (58) ou un revêtement réduisant le frottement est appliqué sur la tête sphérique (20) ou la cavité est revêtue avec un matériau réduisant le frottement.

7. Dispositif pour allonger des parties molles dans la région de la hanche, comprenant :
- un clou médullaire de distraction (12), qui comporte une pièce d'ancrage (14) distale, implantable dans un os de fémur (4) et une pièce télescopique (16) proximale pouvant se déplacer dans le sens longitudinal par rapport à la pièce d'ancrage (14) et dotée d'une extrémité d'appui proximale, sur laquelle une tête sphérique (20) est disposée, et
- le dispositif de support (10) selon l'une des revendications 1 à 6 pour supporter l'extrémité d'appui de la pièce télescopique proximale (16).

8. Dispositif selon la revendication 7, dans lequel le logement sphérique (48) du dispositif de support (10) et le clou médullaire de distraction (12) sont configurés de manière à ce qu'une fois la tête sphérique (20) logée dans le logement sphérique (48), l'axe longitudinal (LT) de la pièce télescopique (16) du clou médullaire de distraction (12) puisse se déplacer dans une région d'un cône imaginaire ayant un angle de cône de 25 à 45°, de préférence d'environ 35° lorsque la pièce d'ancrage (14) est implantée dans l'os du fémur (4) et le dispositif de support (10) est monté sur l'os du bassin (2).
